# EUROPÄISCHE PATENTANMELDUNG

(11) **EP 1 166 719 A2**
(43) Veröffentlichungstag der Anmeldung: **02.01.2002**
(21) Anmeldenummer: 01114078.7
(22) Anmeldetag: 09.06.2001
(51) Int. Cl.: A61B 5/15

(54) **Blutlanzettensystem zur Entnahme von Blut für Diagnosezwecke**

(30) Priorität: 21.06.2000 DE 10030410
(71) Anmelder: Roche Diagnostics GmbH, 68298 Mannheim (DE); F. HOFFMANN-LA ROCHE AG, 4070 Basel (CH)
(72) Erfinder: Kuhr, Hans-Jürgen, 68219 Mannheim (DE); Forster, Richard, 92536 Pfreimd (DE)
(74) Vertreter: Pfeifer, Hans-Peter, Dr.

(57) **Zusammenfassung**

Blutlanzettensystem zur Entnahme von Blut für diagnostische Zwecke mit einem Gehäuse (10), einem in dem Gehäuse (10) beweglichen Lanzettenhalter (12) zur auswechselbaren Halterung einer Lanzette (3) und einem Lanzettenantrieb (15) zum Antreiben der Einstichbewegung des Lanzettenhalters (12) mit der darin gehaltenen Lanzette (3). Das Gehäuse (10) weist an seinem in Einstichrichtung (7) vorderen Ende (21) eine Kappe (20) auf, die abnehmbar ist, um eine verbrauchte Lanzette (3) aus diesem zu entfernen.

Die Lanzette (3) und die Gehäusekappe (20) werden beim Aufsetzen der Gehäusekappe (20) durch einen aufeinander abgestimmte Kopplungselemente (34,35) aufweisenden Kopplungsmechanismus (28) derartig miteinander gekoppelt, daß beim Abnehmen der Gehäusekappe (20) zugleich die Lanzette (3) aus dem Lanzettenhalter (12) herausgezogen wird.

## Beschreibung

Die Erfindung betrifft ein Blutlanzettensystem zur Entnahme von Blut für Diagnosezwecke.

Bei verschiedenen Krankheiten ist es notwendig, das menschliche Blut hinsichtlich eines darin enthaltenen Bestandteiles (Analyten) zu untersuchen. In vielen Fällen reicht es dabei aus, durch Erzeugung einer kleinen Stichwunde dem Körper eine geringe Menge Blut in Form eines Blutstropfens zu entnehmen. Ein besonders wichtiger derartiger Fall ist der Diabetes mellitus, bei dem das Blut in regelmäßigen Abständen auf den Glucosegehalt untersucht werden muß.

Zur Erzeugung der erforderlichen Stichwunde werden üblicherweise Blutlanzettensysteme verwendet, die aus einem Stechgerät und hierzu angepaßten auswechselbaren Lanzetten bestehen. In dem Gehäuse des Stechgerätes befindet sich ein Lanzettenhalter, in den jeweils eine Lanzette auswechselbar eingesetzt werden kann. Beim Einstichvorgang wird der Lanzettenhalter von einem ebenfalls in dem Stechgerät integrierten Lanzettenantrieb mit der Lanzette schnell in eine Einstichrichtung bewegt, bis die Lanzettenspitze aus einer am vorderen Ende des Stechgerätes vorgesehenen Austrittsöffnung austritt und eine kleine Stichwunde in dem Körperteil, gegen das das vordere Ende gedrückt wird, erzeugt. Danach wird der Lanzettenhalter mit der Lanzette entgegen der Einstichrichtung zurückbewegt.

Im Benutzungszustand ist der Lanzettenhalter bei vielen Stechgeräten von einer Gehäusekappe abgedeckt, die das vordere Ende des Gehäuses des Stechgerätes bildet und eine Austrittsöffnung aufweist, durch die die Spitze der Lanzette bei dem Einstichvorgang austreten kann. Die Einstichöffnung ist von einer Andruckfläche umgeben, mit der das Stechgerät bei der Benutzung gegen das Körperteil, aus dem Blut entnommen werden soll (in der Regel die Fingerkuppe oder das Ohrläppchen) gedrückt wird.

Zur Vermeidung von Infektionen sind die Lanzetten zur einmaligen Verwendung vorgesehene disposible Elemente. Um eine neue Lanzette einzusetzen wird die den Lanzettenhalter abdeckende Gehäusekappe abgenommen und die Lanzette in den Lanzettenhalter eingesetzt. Auch zum Entfernen verbrauchter Lanzetten wird die Gehäusekappe abgenommen.

Blutlanzettensysteme werden in großem Umfang nicht nur durch medizinisches Personal, sondern auch durch Laien verwendet. Dies gilt insbesondere für die Therapiekontrolle von Diabetikern. Es wurde festgestellt, daß schwerwiegende mit dem Diabetes verbundene Schäden (zum Beispiel Erblinden) entscheidend vermindert werden können, wenn die Glucosekonzentration im Blut des Diabetikers sehr häufig (vier bis fünf mal täglich) bestimmt und aufgrund dieser Messungen die Insulininjektionen exakt eingestellt werden. Die Bereitschaft und Fähigkeit von Diabetikern, im Rahmen des "Home-monitoring" derartig häufig ihr Blut zu untersuchen hängt wesentlich von der optimalen Funktion des verwendeten Blutlanzettensystems ab.

Ein wesentlicher Teil dieser Funktion betrifft die Handhabungsschritte, die mit dem Einsetzen neuer und Entfernen verbrauchter Lanzetten verbunden sind. Sie sollen einerseits möglichst einfach sein, andererseits aber ein Maximum an Sicherheit gegen Verletzungs- und Infektionsrisiken gewährleisten. Zugleich sollen die Kosten - insbesondere hinsichtlich des disposiblen Verbrauchsmaterials - möglichst gering sein.

Beim Einsetzen der Lanzette ist die scharfe Spitze der Lanzettennadel üblicherweise von einer Spitzenabdeckung aus Kunststoff umgeben, die eine gefahrlose Handhabung ermöglicht. Wenn die Lanzette eingesteckt ist, wird die Spitzenabdeckung entfernt und die Gehäusekappe aufgesetzt. Nach dem Stechvorgang wird die Gehäusekappe abgenommen, so daß die Lanzette wieder zugänglich ist. Sie kann dann manuell entnommen werden, wobei jedoch das Risiko einer Verletzung an der Lanzettenspitze hoch ist, insbesondere wenn man berücksichtigt, daß ein großer Teil der Benutzer durch hohes Alter, schlechte Augen oder Zittern der Hände behindert ist.

Um das Entfernen der verbrauchten Lanzette zu erleichtern, haben manche Geräte einen manuell betätigbaren Auswerfermechanismus. Beispielsweise ist in der US-Patentschrift 5,318,584 ein Blutlanzettensystem beschrieben, bei dem die Blutlanzette mittels einer Auswerferstange aus der Lanzettenhalterung ausgestoßen werden kann, wobei die Auswerferstange durch Drücken eines aus dem hinteren Ende des Stechgerätes hervorstehenden Betätigungsknopfes betätigt wird. Bei dem in der US-Patentschrift 4,442,836 beschriebenen Gerät ist der Auswerfermechanismus so ausgebildet, daß die gebrauchte Lanzette nach jedem Einstichvorgang beim erneuten Spannen des Gerätes automatisch ausgeworfen wird. Derartige Auswerfer-Mechanismen erfordern jedoch einen relativ hohen zusätzlichen konstruktiven Aufwand. Ihre Bedienung ist - unter Berücksichtigung des Gesundheitszustandes vieler Benutzer - nicht so einfach, wie dies wünschenswert wäre.

In der EP 0 958 783 A1 ist ein Stechgerät beschrieben, an dessen Kappe sogenannte Eingriffsmittel (engagement means) vorgesehen sind, die von dem Benutzer aus einer ersten Stellung, in der sie nicht in Eingriff mit der Lanzette stehen, in eine zweite Stellung gebracht werden können, bei der sie die Lanzette kontaktieren und gegen die gegenüberliegende Innenwand der Kappe drücken. Zur Betätigung kann beispielsweise ein Knopf vorgesehen sein, der seitlich aus der Kappe herausragt. Nach dem Einstichvorgang soll der Benutzer auf diesen Knopf drücken, dadurch die Lanzette in der Kappe festklemmen, anschließend die Lanzette und die Kappe gemeinsam abnehmen und schließlich die Lanzette entsorgen. Dies erfordert jedoch einen zusätzlichen diffizilen Handhabungsschritt, der für viele Benutzer ein erhebliches Problem darstellt.

Eine optimale Einfachheit und Sicherheit der Bedienung wird durch Blutlanzettensysteme angestrebt, bei denen die Lanzette in die Kappe integriert ist, so daß die Lanzette und die Gehäusekappe insgesamt eine auswechselbare disposible Einheit bilden. Derartige Konstruktionen sind in der EP-0595148 A1 sowie in den US-Patenten 4,990,154 und 5,628,765 beschrieben. Nachteilig ist dabei jedoch der hohe Materialverbrauch, da nach jeder Benutzung die gesamte disposible Baueinheit einschließlich der Gehäusekappe entsorgt werden muß. Wegen der damit verbundenen hohen Kosten eignen sich diese Geräte nur auf Einsatzgebieten, wo besonders hohe Anforderungen an Sicherheit und Hygiene gestellt werden müssen, insbesondere für die Anwendung im Krankenhaus. Im Bereich des Home-monitoring sind einerseits die Sicherheitsrisiken geringer, weil ein Blutlanzettensystem immer nur von dem gleichen Patienten benutzt wird, andererseits sind die mit integrierten Lanzetten-Kappe-Disposables verbundenen Kosten nicht akzeptabel.

Auf dieser Basis befaßt sich die Erfindung mit dem Problem, insbesondere für den Bereich des Home-monitoring ein Blutlanzettensystem zur Verfügung zu stellen, das mit möglichst geringem Aufwand ein berührungsloses, risikoloses und einfaches Entnehmen verbrauchter Lanzetten aus dem Lanzettenhalter ermöglicht.

Dieses Problem wird bei einem Blutlanzettensystem der vorstehend erläuterten Bauart dadurch gelöst, daß die Lanzette und die Gehäusekappe während des Aufsetzens der Gehäusekappe durch einen aufeinander abgestimmte Kopplungselemente aufweisenden Kopplungsmechanismus derartig miteinander gekoppelt werden, daß beim Abnehmen der Gehäusekappe zugleich die Lanzette aus dem Lanzettenhalter herausgezogen wird.

Im Gegensatz zu den erwähnten Lanzette-Kappe-Disposables ist bei der Erfindung die Lanzette ein separates, von der Gehäusekappe unabhängig hergestelltes und vertriebenes Element. Sie ist das einzige Wegwerfteil. Die Kappe kann beliebig oft verwendet werden und entsprechend hochwertig hergestellt sein. Die Bedienung ist sehr einfach, weil das Abziehen der Gehäusekappe und die Entnahme der gebrauchten Lanzette in einem einzigen Arbeitsschritt erfolgt.

Im Gegensatz zu der in der EP 0 958 783 beschriebenen Konstruktion sind bei der vorliegenden Erfindung keine zusätzlichen Handhabungsschritte erforderlich, weil die Kopplung der Kappe mit der Lanzette automatisch während des Ablaufes der ohnehin erforderlichen und üblichen Handhabungsschritte erfolgt.

Im einfachsten Fall wirkt der Kopplungsmechanismus unmittelbar zwischen der Gehäusekappe und der Lanzette. In der Regel haben Lanzetten einen Lanzettenkörper aus Kunststoff, der die metallische Lanzettennadel umgibt. An diesem Lanzettenkörper kann das erste der aufeinander abgestimmten Kopplungselemente vorgesehen sein. Das zweite Kopplungselement ist vorzugsweise unmittelbar an der Kappe (an deren inneren Wand) angebracht. Beispielsweise kann der Lanzettenkörper eine sich in seiner Längsrichtung erstreckende nutförmige Vertiefung aufweisen, in die eine vorspringende Nase klinkenartig eingreift, die an einer mit der Innenwand der Kappe verbundenen elastischen Zunge hervorspringt. Beim Aufstecken der Kappe rastet die vorspringende Nase in die Nut ein, wobei die Abmessungen der Nut so festgelegt sind, daß die für den Einstichvorgang erforderliche Bewegung der Lanzette in Einstichrichtung möglich ist.

Besonders bevorzugt ist jedoch eine Ausführungsform, bei der der Kopplungsmechanismus ein zusätzliches Bauteil einschließt, das als Auswerfer bezeichnet wird. Der Auswerfer ist in dem Gehäuse so gelagert, daß er in Einstichrichtung verschiebbar ist. Er weist ein Lanzettenkontaktelement auf, das die Lanzette beim Auswerfen kontaktiert und dadurch aus der Halterung herausdrückt oder -zieht. Bei dieser Ausführungsform ist ein erstes der aufeinander abgestimmten Kopplungselemente an dem Auswerfer und vorzugsweise ein zweites der aufeinander abgestimmten Kopplungselemente an der Kappe angeordnet.

Die Erfindung wird nachfolgend anhand eines in den Figuren dargestellten Ausführungsbeispieles näher erläutert. Die darin beschriebenen Besonderheiten können einzeln oder in Kombination miteinander eingesetzt werden, um bevorzugte Ausgestaltungen der Erfindung zu schaffen.

### Es zeigen:

- Fig. 1: Eine perspektivische Explosionsdarstellung einer Lanzette und des vorderen Teils eines Stechgerätes,
- Fig. 2: einen Längsschnitt durch das Stechgerät im Benutzungszustand (Lanzette eingesetzt, Auswerfer eingeschoben),
- Fig. 3: einen Längsschnitt entsprechend Fig. 2 während des Abnehmens der Kappe (Lanzette frei, Auswerfer ausgezogen),
- Fig. 4: eine perspektivische Darstellung des Lanzettenhalters, der Lanzette und des Auswerfers in der Position gemäß Fig. 2,
- Fig. 5: eine perspektivische Darstellung entsprechend Fig. 4 ohne den Lanzettenhalter,
- Fig. 6: einen Längsschnitt entsprechend Fig. 2 in verkleinerter Darstellung in einer Benutzungsphase, bei der gerade eine unbenutzte Lanzette eingesteckt wurde,
- Fig. 7: einen Längsschnitt entsprechend Fig. 6 in einer Benutzungsphase, bei der die Gehäusekappe aufgesteckt wird,
- Fig. 8: einen Längsschnitt entsprechend Fig. 6 in einer Benutzungsphase, bei der die Gehäusekappe abgenommen wird,
- Fig. 9: einen Längsschnitt entsprechend Fig. 8 in einer etwas späteren Benutzungsphase beim Abnehmen der Gehäusekappe.

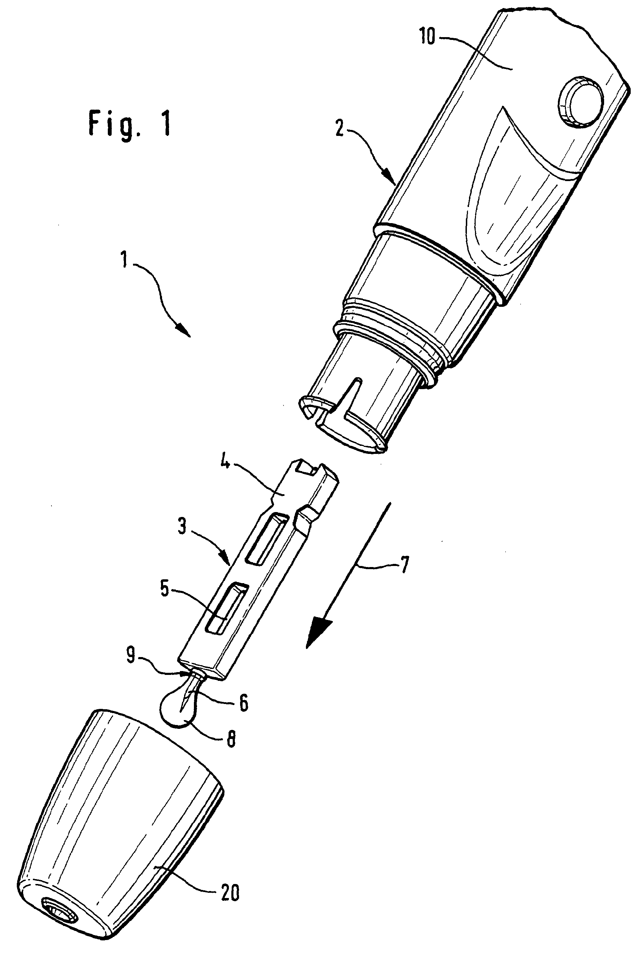

Das in den Figuren 1 bis 5 dargestellte Blutlanzettensystem 1 besteht aus einem Stechgerät 2 und zur Verwendung mit dem Stechgerät 2 angepaßten auswechselbaren Lanzetten 3. Die Lanzetten 3 haben jeweils einen Lanzettenkörper 4 aus Kunststoff und eine in dem Lanzettenkörper 4 fixierte Nadel 5, deren scharfe Spitze 6 in der durch den Pfeil 7 symbolisierten Einstichrichtung nach vorne aus dem Lanzettenkörper herausragt. Bei der in Figur 1 dargestellten unbenutzten Lanzette 3 ist die Spitze 6 von einer Spitzenabdeckung 8 umhüllt, die mit dem Lanzettenkörper 4 über eine Sollbruchstelle 9 so verbunden ist, daß sie leicht durch Drehen und Ziehen entfernt und die Spitze 6 freigelegt werden kann.

Bei der dargestellten bevorzugten Ausführungsform ist das Gehäuse 10 des Stechgerätes 2 langgestreckt ausgebildet, so daß seine äußere Form einem Kugelschreiber ähnlich ist (sogenannte "pencil-Bauform"). Die Längsachse 11 des Stechgerätes 2 verläuft in Einstichrichtung 7.

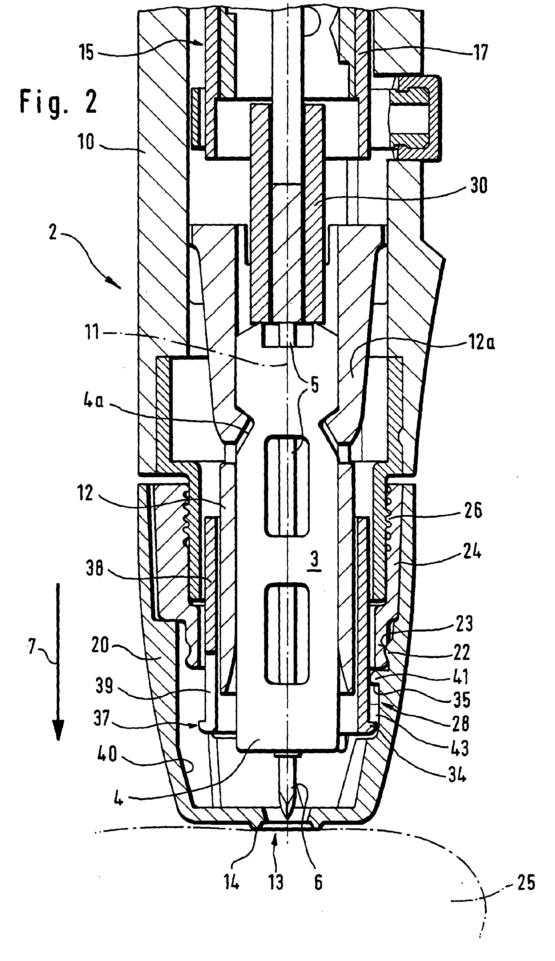

In dem Gehäuse 10 befindet sich ein Lanzettenhalter 12. Die Lanzette 3 ist in dem Lanzettenhalter 12 mittels einer geeigneten Klemmbefestigung in einer genau reproduzierbaren Längsposition fixiert. Im dargestellten Fall weist der Lanzettenhalter 12 zwei nach radial innen vorgespannte Haltezungen 12a auf, die in entsprechende Ausnehmungen 4a des Lanzettenkörpers 4 eingreifen. Nähere Einzelheiten zu dieser Befestigungsart sind dem US-Patent 5,318,584 zu entnehmen. Der Lanzettenhalter ist derartig geführt und gelagert, daß er mit möglichst geringen Vibrationen innerhalb des Gehäuses 10 in Einstichrichtung 7 nach vorne bewegt werden kann, bis die Lanzettenspitze 6 aus einer Austrittsöffnung 13 austritt, um eine Stichwunde in einem an einer die Austrittsöffnung 13 ringförmig umgebenden Andruckfläche 14 anliegenden Körperteil 25 (Fig. 2) zu erzeugen.

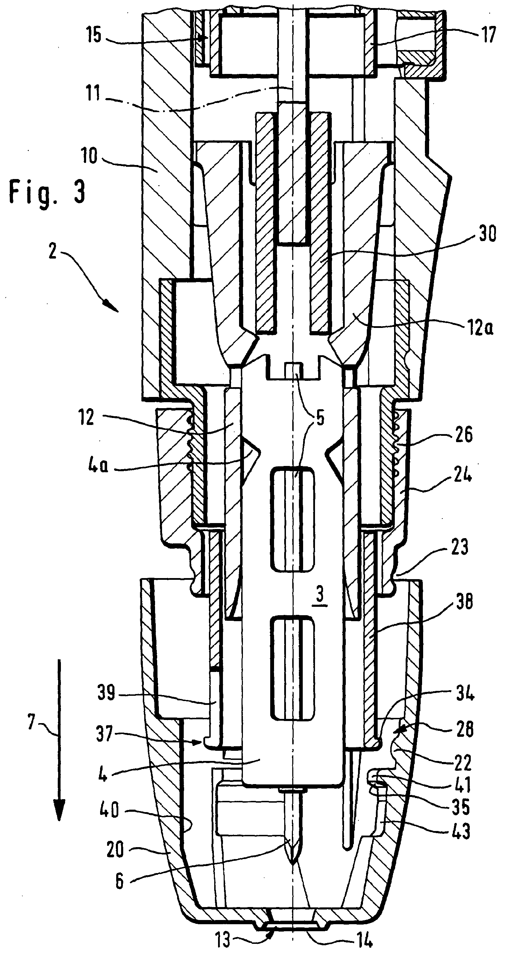

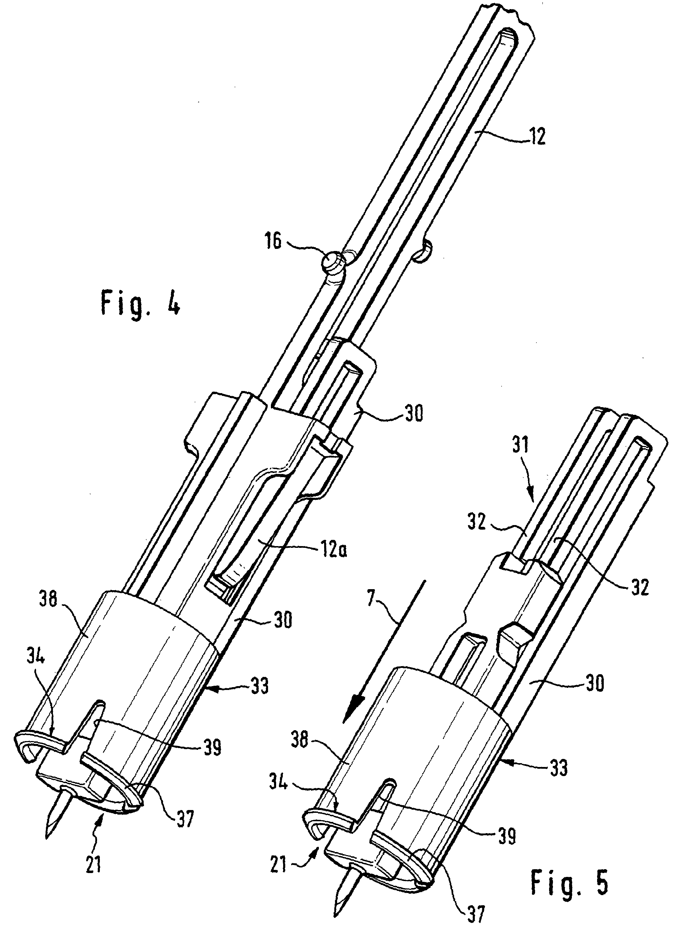

Die Einstichbewegung des Lanzettenhalters 12 und einer darin gehaltenen Lanzette 3 wird von einem insgesamt mit 15 bezeichneten Lanzettenantrieb angetrieben. Bevorzugt ist der Lanzettenantrieb gemäß dem in der US-Patentschrift 5,318,584 beschriebenen Prinzip realisiert. Dabei wird eine Kurvensteuerung verwendet. An dem Lanzettenhalter ist ein Steuerzapfen 16 (Fig. 4) vorgesehen, der in eine passende als Steuerkurve wirkende Ausnehmung eines Antriebsrotors 17 (nur in den Figuren 2 und 3 teilweise erkennbar) eingreift, der eine Drehung um die Längsachse 11 des Gehäuses 10 ausführt. Die Rotation des Antriebsrotors 17 und der darin vorgesehenen Steuerkurve führt zu einer entsprechenden Längsverschiebung des Steuerzapfens 16 und somit des Lanzettenhalters 12 in Einstichrichtung 7. Nähere Einzelheiten über diesen bevorzugten Lanzettenantrieb 15 können der genannten US-Patentschrift entnommen werden.

Das in Einstichrichtung 7 vordere Ende des Gehäuses 10 wird von einer Kappe 20 gebildet, die das zum Einführen der Lanzette 3 vorgesehene Einführende 21 des Lanzettenhalters 12 abdeckt. Die Kappe 20 ist abnehmbar an dem Gehäuse 10 befestigt, wobei unterschiedliche Befestigungsprinzipien verwendet werden können, sofern sichergestellt ist, daß die Längsposition der Gehäusekappe 20 relativ zu dem Lanzettenhalter 12 präzise und reproduzierbar definiert ist. Bei der dargestellten Ausführungsform wird eine Schnappbefestigung verwendet, bei der ein an der Kappe 20 vorgesehener Wulst 22 in eine entsprechende Vertiefung 23 eines Gehäuseteils 24 eingreift. Statt einer solchen Schnappbefestigung ist beispielsweise auch eine Gewindebefestigung geeignet.

Das Gehäuseteil 24, an dem die Gehäusekappe 20 befestigt wird, ist bei der dargestellten bevorzugten Ausführungsform in seiner Längsposition mittels eines Schraubgewindes 26 relativ zu dem übrigen Gehäuse 10 und damit relativ zu dem Lanzettenhalter 12 verstellbar. Dadurch ist eine Einstellung der Einstichtiefe möglich. Je mehr das Gehäuseteil 24 und damit die Kappe 20, an der sich die Andruckfläche 14 befindet, in Einstichrichtung 7 nach vorne verstellt werden, desto geringer wird bei einer vorgegebenen in Einstichrichtung 7 untersten Position des Lanzettenhalters 12 die Einstichtiefe.

Ein Kopplungsmechanismus 28, durch den die Lanzette 3 und die Gehäusekappe 20 beim Aufsetzen der Gehäusekappe auf das Gehäuse 10 miteinander gekoppelt werden, wird mittels eines in Einstichrichtung 7 verschiebbar gelagerten Auswerfers 30 gebildet, der in seiner Gesamtheit am besten in Figur 5 zu erkennen ist. Generell wird der Auswerfer 30 von einem Konstruktionsteil gebildet, in dessen in Einstichrichtung vorderem Bereich ein Kopplungselement 34 zur Kopplung mit der Gehäusekappe 20 und in dessen in Einstichrichtung hinterem Bereich ein Lanzettenkontaktelement 31 vorgesehen ist, das derartig mit der Lanzette 3 in Kontakt steht, daß eine Verschiebung des Lanzettenkontaktelementes 31 in Einstichrichtung 7 dazu führt, daß die Lanzette 3 aus dem Lanzettenhalter 12 ausgestoßen wird. Der zwischen dem Lanzettenkontaktelement 31 und dem Kopplungselement 34 liegende Teil des Auswerfers 30 wird als Mittelteil 33 bezeichnet.

Im dargestellten Fall wird das Lanzettenkontaktelement 31 durch zwei Ausstoßstangen 32 gebildet, die gegen das hintere Ende der Lanzette 3 stoßen. Ein Kopplungselement 34 des Auswerfers 30 und ein zugehöriges Kopplungselement 35 der Gehäusekappe 2 sind wechselseitig derartig aufeinander abgestimmt, daß die Kappe 20 mit dem Auswerfer 30 und somit indirekt mit der Lanzette 3 gekoppelt wird, wenn die Gehäusekappe 20 auf das Gehäuse 10 aufgesetzt wird. Im dargestellten Fall besteht das auswerferseitige Kopplungselement 34 aus drei Segmenten eines nach radial außen vorstehenden Ringkragens 37, der an einer die Lanzettenführung umgebenden Hülse 38 ausgebildet ist. Die Hülse 38 weist im Bereich des Kragens 37 Einschnitte 39 auf, die die Segmente des Ringkragens 37 voneinander trennen und eine elastische Deformation der Hülse 38 im Bereich des Ringkragens 37 nach radial innen ermöglichen.

Das an der Gehäusekappe 20 vorgesehenen Kopplungselement 35 wird bei der dargestellten Ausführungsform durch einen an deren Innenwand 40 vorgesehenen ringförmigen Vorsprung 41 gebildet, der so angeordnet und dimensioniert ist, daß beim Aufstecken der Kappe 20 die Hülse 38 im Bereich des Ringkragens 37 leicht zusammengedrückt wird und der Ringkragen 37 nach Passieren der durch den ringförmigen Vorsprung 41 gebildeten Engstelle in eine in Einstichrichtung 7 vor dem ringförmigen Vorsprung 41 vorgesehene Vertiefung 43 eindringt.

Es sind zahlreiche Variationen des Kopplungsmechanismus 28 möglich, sofern gewährleistet ist, daß einerseits die Kopplung zwischen der Gehäusekappe 20 und der Lanzette 3 (vorzugsweise indirekt über einen Auswerfer 30) erst beim Aufstecken der Gehäusekappe 20 auf das Gehäuse 10 hergestellt wird (nachdem zuvor die Lanzette 3 als separates Teil in den Lanzettenhalter 12 eingesetzt wurde) und daß beim Abnehmen der Kappe 20 zugleich die Lanzette 3 aus dem Lanzettenhalter 12 herausgezogen wird. Das dargestellte Kopplungsprinzip, bei dem mindestens eines der Kopplungselemente 34,35 derartig elastisch gelagert ist, daß es bei dem Kopplungsvorgang klinkenartig in eine entsprechende Ausnehmung (hier die Vertiefung 43) des anderen Kopplungselementes (hier 35) eingreift, ist bevorzugt, weil sie eine besonders einfache Bedienung erlaubt. Eine geeignete Kopplung kann aber beispielsweise auch mittels einer Bajonettverbindung hergestellt werden.

Auch der für das Entfernen der Lanzette 3 aus dem Lanzettenhalter 12 erforderliche Kontakt zwischen einem an dem Auswerfer 30 vorgesehenen Lanzettenkontaktelement 31 und der Lanzette muß nicht notwendigerweise mittels eines Ausstoßers realisiert sein, der - wie die Ausstoßstange 32 - von hinten gegen die Lanzette drückt. Vielmehr ist auch eine Konstruktion möglich, bei der die Lanzette 3 aus dem Lanzettenhalter 12 herausgezogen wird, wobei ein an einem entsprechend gestalteten Auswerfer vorgesehenes Lanzettenkontaktelement zweckmäßigerweise von der Seite her in eine entsprechende Ausnehmung des Lanzettenkörpers eingreift.

Die dargestellte Ausführungsform, bei der der Mittelteil 33 des Auswerfers 30, der das auswerferseitige Kopplungselement 34 mit dem Lanzettenkontaktelement 31 verbindet, zumindest teilweise hülsenförmig ausgebildet ist, ist besonders vorteilhaft, weil die Hülse 38 eine präzise Führung des darin befindlichen Lanzettenhalters 12 ermöglicht. Auch insoweit sind jedoch andere Gestaltungen möglich. Beispielsweise kann der Auswerfer im wesentlichen von zwei oder mehreren in Einstichrichtung verlaufenden, vorzugsweise rotationssymmetrisch angeordneten Stangen gebildet werden, an deren vorderem Ende Kopplungselemente zur Kopplung mit der Kappe 20 und in deren hinteren Bereich ein Lanzettenkontaktelement 31 vorgesehen ist.

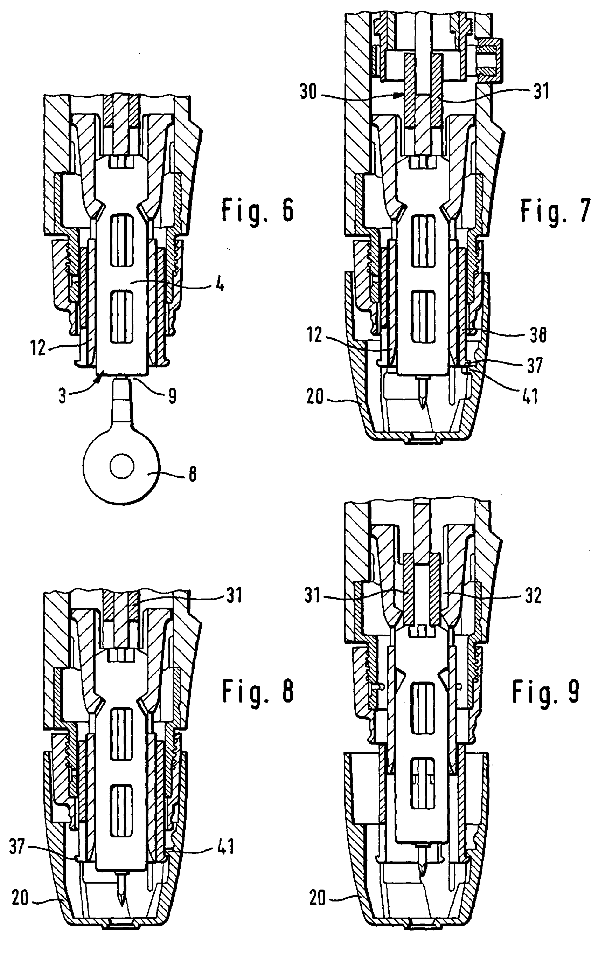

Die Figuren 6 bis 9 zeigen ergänzend zu den Figuren 2 und 3 einige weitere Benutzungsphasen des erfindungsgemäßen Blutlanzettensystems 1.

die Lanzette wird an der Spitzenabdeckung 8 ergriffen und bei abgenommener Gehäusekappe 20 in die Halterung 12 eingesetzt (Fig. 6). Danach wird die Spitzenabdeckung 8 abgedreht und die Gehäusekappe 20 aufgesetzt, wobei deren Vorsprung 41 gegen den Ringkragen 37 der Hülse 38 drückt und dadurch den Auswerfer 30 nach hinten verschiebt, bis seine axiale Rückwärtsbewegung durch einen Anschlag begrenzt wird (Fig. 7).

Wenn die Gehäusekappe 20 weiter nach hinten (in die in Fig. 2 dargestellte Position) geschoben wird, gelangt der ringförmige Vorsprung 41 in eine Position axial hinter dem Ringkragen 37, so daß die Kopplungselemente 34,35 miteinander in Eingriff sind.

Nach dem Einstichvorgang, der sich nicht von vorbekannten Blutlanzettensystemen unterscheidet, wird die Gehäusekappe 20 wieder abgenommen. Dabei gelangt sie in die in Fig. 8 dargestellte Position, bei der der ringförmige Vorsprung 41 an dem Ringkragen 37 anliegt. Dadurch wird bei weiterem Bewegen der Gehäusekappe 20 nach vorne (in Einstichrichtung 7) der Auswerfer 30 nach vorne verschoben, wobei die Lanzette 3 durch dessen Lanzettenkontaktelement 31 (Ausstoßstange 32) nach vorne aus der Halterung herausgedrückt wird. Die Lanzette fällt dann in die Gehäusekappe 20 oder verbleibt lose im unteren Bereich des Gehäuses 10. Nach dem Abnehmen der Gehäusekappe 20 kann die verbrauchte Lanzette 3 in einen Abfallbehälter gekippt werden, ohne daß sie berührt werden muß.

## Patentansprüche

1. Blutlanzettensystem (1) zur Entnahme von Blut für diagnostische Zwecke, umfassend
Lanzetten (3) und
ein Stechgerät (2) mit einem Gehäuse (10), einem in dem Gehäuse (10) beweglichen Lanzettenhalter (12) zur auswechselbaren Halterung einer Lanzette (3) und einem Lanzettenantrieb (15) zum Antreiben der Einstichbewegung der Lanzette (3) auf einem vorbestimmten Einstichweg,
wobei das Gehäuse (10) an seinem in Einstichrichtung (7) vorderen Ende (21) eine Kappe (20) aufweist, die abnehmbar ist, um eine verbrauchte Lanzette (3) aus dem Gehäuse (10) zu entfernen,
**dadurch gekennzeichnet, daß**
die Lanzette (3) und die Gehäusekappe (20) beim Aufsetzen der Gehäusekappe (20) durch einen aufeinander abgestimmte Kopplungselemente (34,35) aufweisenden Kopplungsmechanismus (28) derartig miteinander gekoppelt werden, daß beim Abnehmen der Gehäusekappe (20) zugleich die Lanzette (3) aus dem Lanzettenhalter (12) herausgezogen wird.

2. Blutlanzettensystem nach Anspruch 1, **dadurch gekennzeichnet, daß** sie einen in dem Gehäuse (10) in Einstichrichtung (7) verschiebbar gelagerten Auswerfer (30) zum Auswerfen der Lanzette (3) aus dem Lanzettenhalter (12) aufweist, der ein Bestandteil des Kopplungsmechanismus (28) ist und an dem einerseits ein erstes (34) der aufeinander abgestimmten Kopplungselemente und andererseits ein Lanzettenkontaktelement (31), das bei dem Auswerfvorgang die Lanzette (3) kontaktiert, angeordnet ist.

3. Blutlanzettensystem nach Anspruch 2, **dadurch gekennzeichnet, daß** ein zweites (35) der aufeinander abgestimmten Kopplungselemente an der Gehäusekappe (20) angeordnet ist.

4. Blutlanzettensystem nach Anspruch 2 oder 3, **dadurch gekennzeichnet, daß** der Auswerfer (30) einen in einer Führung parallel zu dem Einstichweg verschiebbaren Mittelteil (33) aufweist, dessen vorderes Ende mit dem Kopplungselement (34) und dessen hinteres Ende mit dem Lanzettenkontaktelement (31) verbunden ist.

5. Blutlanzettensystem nach Anspruch 4, **dadurch gekennzeichnet, daß** der verschiebbare Mittelteil (33) eine die Lanzettenführung umgebende Hülse (38) aufweist, die eine in den Lanzettenhalter (12) eingesetzte Lanzette (3) umgibt.

6. Blutlanzettensystem nach einem der vorhergehenden Ansprüche, **dadurch gekennzeichnet, daß** mindestens eines der Kopplungselemente (34,35) einen quer zu der Einstichrichtung elastisch gelagerten Vorsprung (41) aufweist, der bei dem Kopplungsvorgang klinkenartig in eine entsprechende Ausnehmung (43) des anderen Kopplungselementes eingreift.

7. Blutlanzettensystem nach einem der vorhergehenden Ansprüche, **dadurch gekennzeichnet, daß** die Lanzette einen Lanzettenkörper (4) aus Kunststoff, eine in dem Lanzettenkörper (4) fixierte Lanzettennadel (5) und eine die Lanzettenspitze (6) umgebende Spitzenabdeckung (8) aufweist, die mit dem Lanzettenkörper (4) über eine Sollbruchstelle (9) verbunden und nach dem Einsetzen der Lanzette (3) in den Lanzettenhalter (12) abtrennbar ist, um die aus dem Lanzettenkörper (4) herausragende Lanzettenspitze (6) freizulegen.

8. Blutlanzettensystem nach einem der vorhergehenden Ansprüche, **dadurch gekennzeichnet, daß** die Gehäusekappe (20) eine Andruckfläche (14) zum Andrücken an die Haut bei der Benutzung des Blutlanzettensystems (1) aufweist, die Gehäusekappe (20) an dem Gehäuse (10) in einer in Einstichrichtung exakt definierten Position fixierbar ist und eine Einstellvorrichtung (26) vorgesehen ist, durch die zur Einstellung der Einstechtiefe die Längsposition der Kappe (20) in Richtung der Einstichrichtung verstellbar ist.
